# EUROPEAN PATENT SPECIFICATION

(11) **EP 2 284 284 B1**
(45) Date of publication and mention of the grant of the patent: **25.02.2015**
(21) Application number: 09172821.2
(22) Date of filing: 13.10.2009
(51) Int. Cl.: G01N 33/543, G01N 33/542

(54) **Antigen detection kit and method**
Antigen-Detektionskit und -verfahren
Kit de détection d'antigènes et procédé

(30) Priority: 30.07.2009 KR 20090070041
(43) Date of publication of application: 16.02.2011
(73) Proprietor: Korea Institute of Science and Technology, Seoul 136-791 (KR)
(72) Inventor: Ahn, Dae-Ro, Seoul 136-130 (KR); Yang, Eun-Gyeong, Seoul 137-948 (KR); Han, Ki-Cheol, Seoul 136-130 (KR)
(74) Representative: Delorme, Nicolas

(56) References cited:
- WO-A1-94/26932
- WO-A1-2008/111818
- US-A1- 2002 051 974
- AHN DAE-RO ET AL: "An RNase H-assisted fluorescent biosensor for aptamers" CHEMBIOCHEM - A EUROPEAN JOURNAL OF CHEMICAL BIOLOGY, WILEY VCH, WEINHEIM, DE, vol. 8, no. 12, 17 July 2007 (2007-07-17), pages 1347-1350, XP002486877 ISSN: 1439-4227
- MALLET F ET AL: "Enzyme-linked oligosorbent assay for detection of polymerase chain reaction-amplified human immunodeficiency virus type 1." JOURNAL OF CLINICAL MICROBIOLOGY JUN 1993, vol. 31, no. 6, June 1993 (1993-06), pages 1444-1449, XP002559304 ISSN: 0095-1137

### Sequence Listing

<110> Korea Institute of Science and Technology
<120> Antigen Detecting Kit and Method
<130> OPP20091490US
<160> 4
<170> Kopatentin 1.71
<210> 1
   <211> 30
   <212> DNA
   <213> Artificial sequence
<220>
   <223> DNA oligonucleotide to be conjugated to detection antibody for carcinoembryonic antigen
<400> 1
   tttttttttt tttttttttt aaccacagtg 30
<210> 2
   <211> 10
   <212> RNA
   <213> Artificial Sequence
<220>
   <223> RNA oligonucleotide for carcinoembryonic antigen
<400> 2
   cacugugguu 10
<210> 3
   <211> 30
   <212> DNA
   <213> Artificial sequence
<220>
   <223> DNA oligonucleotide to be conjugated to detection antibody for Prostate specific antigen
<400> 3
   tttttttttt tttttttttt actctatggg 30
<210> 4
   <211> 10
   <212> RNA
   <213> Artificial sequence
<220>
   <223> RNA oligonucleotide for Prostate specific antigen
<400> 4
   cccauagagu 10

## Description

### BACKGROUND OF THE INVENTION

### (a) Field of the Invention

The invention provides a kit for detecting an antigen comprising a capture antibody, a detection antibody bound to a single stranded DNA oligonucleotide, a single stranded RNA oligonucleotide which is complementary to the DNA oligonucleotide, and an RNase; and a method for detecting an antigen using the same. The present invention achieves an immunoassay for multiple biomaterials, with a single analytic tool and a single RNase.

### (b) Description of the Related Art

ELISA (Enzyme-Linked Immunosorbent Assay) is an immunoassay, which detects a specific material to be analyzed by using the reaction of the material with an antibody corresponding to the material. Before development of ELISA, the only option for conducting an immunoassay was radioimmunoassay, a technique using a radioactively-labeled antigen or antibody. Because radioactivity poses a potential health threat, an alternative immunoassay, which use a non-radioactive signal in place of radioactive signals, was demanded. In respond to the demand, ELISA has been developed and become one of the most commonly performed immunoassays high sensitivity to detect the presence of various target substances.

Generally, ELISA involves a step of detecting a target substance by using an enzyme-labeled antibody and a substrate that reacts with the enzyme to generate signals. The enzymatic reaction leads to a change in color or fluorescence. Since ELISA should be separately performed for each substance when used for analysis of more than one substance in a sample, it is difficult to directly compare relative amounts of various substances coexisting in the sample.

One difference between ELISA and other immunoassays is presence/absence of a signal amplifying process. Most immunoassays do not involve a process of signal amplification by an enzyme, and are generally based on fluorescent signals. Immunoassays utilizing signals of fluorophores labeled on antibodies can simply be used to perform a multiplex immunoassay, because the emission of each of different fluorophores at a distinct wavelength can be differentially used for analysis of multiple substances.. In contrast, an enzyme-based immunoassay such as ELISA, utilizing changes in absorbance or fluorescence from the enzymatic reaction, needs different enzyme-antibody conjugates for the multiplex assay and requires identification and use of multiple pairs of enzyme-substrate. Thus, multiplexing ELISA becomes technically more difficult to achieve than other immunoassays as the number of analytes in the sample increases.

The most widely known multiplex immunoassay without enzymatic amplification of signals uses different kinds of antibodies respectively capable of specific bindings to corresponding target molecules are immobilized on the microsphere phase, which contains different fluorescence substances respectively corresponding to the target molecules. Then fluorescence signals are measured for the microspheres in which the antibodies are bound to the target molecules. However, this method requires the use of an expensive instrument called flow cytometry and is thus less cost-effective than ELISA, which is readily accessible in many labs. Recently, some multiplex immunoassays performable on a single microplate have been introduced. In one method, for example, anti-IgGs are immobilized on about 6-micrometer polystyrene beads and are allowed to bind to capture antibodies. To perform a multiplex assay, detection antibodies for different kinds of analytes are added with different fluorescent substances. In another method called multiplex-fLISA (fluorescence-linked immunosorbent assay), antibodies to multiple analytes are linked to different QDots each emitting at a different wavelength. Immunoassays for multiple analytes in the same sample can be performed at a time through the analysis of fluorescence intensity. Those methods have a drawdack in that their detection limit is less sensitive, compared to that of ELISA, as they do not include the process of signal amplification by enzyme. GenTel provides a multiplex immunoassay in which each of multiple antibodies is directly bound to a different fluorescent substance, and is measured for fluorescent intensity.

ELISA-based multiplex immunoassays using enzymatic reactions, include a method as provided by Quansys Biosciences, Inc. Under this method, small 20 nano-liter spots are deposited on the bottom of a well in the 96-well plate, each spot representing the analytic result for a single target substance. The amount of luminescence increased by peroxidase-substrate reaction is quantified by reading the luminescence image, such that a multiplex-ELISA format is provided. This method has drawbacks; it needs pre-patterned spots on the plate and an expensive instrument to acquire and analyze images. Another ELISA-based multiplex immunoassay uses two antibodies respectively linked to alkaline phosphatase and peroxidase in order to measure two kinds of analytes in the same sample. The enzymes provide the effect of signal amplification. While this method is in principle considered as a multiplex ELISA, it practically becomes more difficult to perform as the number of analytes to be analyzed in the sample increases above two. Moreover, it is not cost-effective due to the use of different kinds of enzymes. Hence, a novel multiplex immunoassay needs to be developed which can be performed in a more convenient manner.

### SUMMARY OF THE INVENTION

The inventors designed an immunoassay which can be applied to multiplex analysis, using only one enzyme, an antibody-coated support, and absorption or fluorescence meauring instruments as having been used in the conventional ELISA.

Therefore, an embodiment provides a kit for detecting an antigen, which includes a capture antibody, a detection antibody conjugated with a single stranded DNA oligonucleotide, a single stranded RNA oligonucleotide which is complementary to the DNA oligonucleotide and labeled with a fluorescent substance, and an RNase.

Another embodiment provides a method of detecting an antigen, which uses a capture antibody, a detection antibody conjugated with a single stranded DNA oligonucleotide, a single stranded RNA oligonucleotide which is complementary to the DNA oligonucleotide and labeled with a fluorescent substance, and an RNase.

### BRIEF DESCRIPTION OF THE DRAWINGS

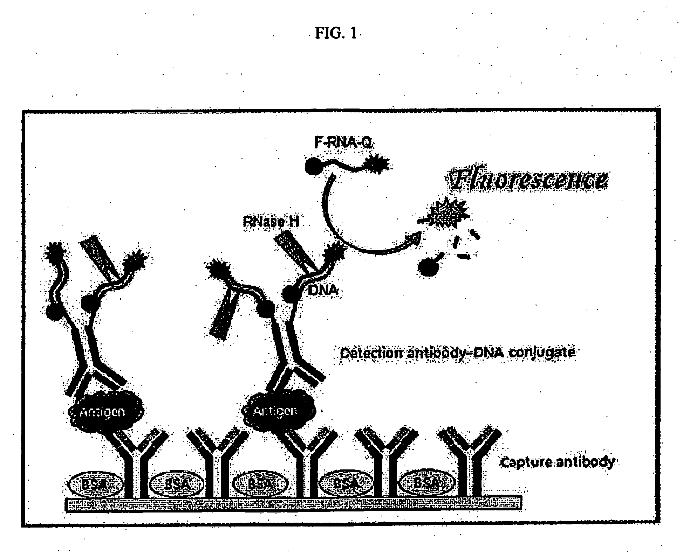

Fig. 1 shows a schematic view of multiplex OLISA (oligonucleotide-linked immunosorbent assay).
Fig. 2 is a graph for detection of AFP (Alpha-fetoprotein), a marker for liver cancer, as obtained using OLISA.
Fig. 3 is a graph for simultaneous detection of AFP (Alpha-fetoprotein), a marker for liver cancer, and PSA (Prostate specific antigen), a marker for prostatic carcinoma, as obtained using multiplex OLISA.

### DETAILED DESCRIPTION OF THE EMBODIMENTS

The present invention relates to technology which enables a novel multiplex immunoassay using antibodies, based on a modifcation of the preexisting form of ELISA.

The technology according to the present invention uses a DNA oligonucleotide conjugated-antibody instead of a peroxidase-fused antibody, for the purpose of signal amplification by enzyme. Further, it uses a RNA oligonucleotide which is complementary to the DNA oligonucleotide and labeled with fluorescent substance at one terminus and fluorescence quenching substance at the other terminus, and an RNase (i.e., RNase H), so that fluorescence signals are amplified by RNA degradation by RNase H when a target molecule exists. Since oligonucleotide, rather than an enzyme, is conjugated to antibodies in the technology according to the present invention, the technology is referred to 'OLISA (oligonucleotide-linked immunosorbent assay)' herein.

Using the OLISA technology, one or more kinds of target molecules (antigens) existing in the same sample can be simultaneously detected. It is possible to detect multiple target molecules by using antibodies which are respectively specific to the two or more target molecules antigens and are respectively conjugated DNA oligonucleotides of different sequences, and using the RNA oligonucleotides which are respectively complementary to the each DNA oligonucleotide and labeled with fluorescent substance at one terminus and fluorescence quenching substance at the other terminus. With signal amplification by RNase H, the target molecules can be analyzed at a time. That is, the technology of the present invention makes it possible that signal amplification process is performed by a single kind of enzyme, and different kinds of antigens (target molecules) in the same sample are simultaneously analyzed (multiplex immunoassay).

An embodiment provides a novel immunoassay which uses an oligonucleotide. In other words, the embodiment provides an OLISA (oligonucleotide-linked immunosorbent assay)-based immunoassay. An assay according to the embodiment contains the step of immunoassaying by amplifying detection signals using a detection antibody conjugated to a DNA oligonucleotide (hereinafter, d-Ab-DNA), an RNA oligonocleotide (hereinafter, F-RNA-Q) which is complementary to the DNA oligonucleotide and labeled with a fluorescent substance (fluorophore, F) at one end and a fluorescence quenching substance (quencher, Q) at the other end, and an RNase. Principles, on which the OLISA immunoassay is based, and methods to perform it, as well as multiplex-OLISA methods and multiplex-OLISA kits, are provided.

The following provides the detailed description of the present invention.

An embodiment provides an immunoassay-based target material (antigen) detection kit for detecting one or more kinds of target materials in a sample.

More specifically, the detection kit basically contains a support and a capture antibody (c-Ab) which is immobilized to the support and capable of specifically binding to a target material (antigen) in a sample. The support may be of any type of solid supports as conventionally used in solid-based immunoassays or a microtiter plate well as commonly used in ELISA. Examples of the support may include, but not be limited to, a flat bottom plate, a U bottom plate, depending on the shapes thereof; an avidin-coated plate, a streptavidin-coated plate, a glutathione coated plate, an anti-GST coated plate depending on the presence of ligand or protein coating; a polystyrene plate, a polypropylene plate, depending on the material used for the support. The capture antibody maybe immobilized to the support by any manner as used for immunoassay purposes, without being limited to a particular method. For example, in an embodiment, the capture antibody may be coated on the support.

The method of immobilizing one part of the antigen-antibody pair to a solid phase can be effective for detection using liquid samples such as body fluid, compared to the case in which detection is performed on mixtures in liquid phase. That is because, where detection is performed in liquid phase, degradation of F-RNA-Qs by an RNase present in the body fluid can occur irrespective of their binding with DNAs and, as a result, may generate false-positive signals. However, occurrence of false-positive signals significantly can be decreased when antigen detection is performed using antibodies immobilized on a solid support, as in ELISA and OLISA, because an RNase H and F-RNA-Qs are added to generate signals once after antigen-antibody interactions are allowed and then, non-reacted fluids and otherwise substances that may inhibit RNase-based assays are removed by washing.

When the kit is exposed to a sample and allowed for interaction for sufficient time, an antigen specific to the antibody in the sample binds to the capture antibody. Subsequently, a d-Ab-DNA conjugate (in which a detection antibody capable of specifically binding to the antigen is conjugated with a single stranded DNA oligonucleotide) is applied on the kit. Then the detection antibody binds to a different site of the antigen from the site of antigen to which the capture antibody binds, resulting in a complex of the capture antibody-antigen-detection antibody-DNA oligonucleotide conjugate. Next applied on the kit is an F-RNA-Q conjugate which contains a single RNA oligonucleotide that is complementary to the DNA oligonucleotide of the d-Ab-DNA and labeled with a fluorescent substance at one end and fluorescence quenching substance at the other end. When the DNA oligonucleotide and the RNA oligonucleotide forms a double stranded DNA/RNA complementary complex, a complex of capture antibody-antigen-detection antibody-DNA/F-RNA-Q is generated. That is, the DNA oligonucleotide conjugated with the detection antibody replaces the role of a detection antibody in ELISA.

When the double stranded DNA/RNA complex is treated with an RNase (e.g., RNase H), the RNA in the complex is degraded to release the fluorescent substance, thereby generating fluorescence. By measuring the intensity of the fluorescence, determination of antigen existence and quantitative analysis can be achieved. As used herein, the term 'antigen detection' may refer to the determination of existence and/or quantity of the target antigen.

Therefore, the kit may further contain a detection antibody-DNA oligonucleotide conjugate, a fluorescent substance-RNA-fluorescence quenching substance, and an RNase, in addition to the support and the capture antibody. They all can be included along with the support and the capture antibody in a single unit. Alternatively, only some of them can be included with the support and the capture antibody in a single unit, with remainders provided in a separate unit. Still alternatively, all or each of them can be provided in a unit separate from the support and the capture antibody.

Another embodiment provides an antigen detection method for a specific antigen (target material) in a sample. In particular, the method may include the following steps of:
contacting a sample with a capture antibody (c-Ab) capable of specifically binding to an antigen to be detected, allowing an antigen-antibody interaction, so that the antigen in the sample binds to the c-Ab (Step (1));
contacting a detection antibody (d-Ab)-DNA oligonucleotide conjugate, where a detection antibody capable of specifically binding to the antigen and a single stranded DNA oligonucleotide are conjugated, with the antigen bound to the capture antibody, wherein the sites of antigen to which the capture antibody binds and the detection antibody binds are different from each other, so that the antigen binds to the d-Ab-DNA oligonucleotide conjugate to a c-Ab/antigen/d-Ab-DNA oligonucleotide conjugate (Step (2));
contacting a F-RNA-Q, which is constructed from a RNA oligonucleotide complementary to the DNA oligonucleotide, a fluorescent substance (fluorophore, F) at one terminus of the RNA oligonucleotide and fluorescence quenching substance (quencher, Q) at the other terminus of the RNA oligonucleotide, with the c-Ab/antigen/d-Ab-DNA oligonucleotide conjugate, forming a DNA-RNA double strand through the hybridization of the DNA and the RNA (Step (3));
treating the DNA-RNA double strand with an RNase which specifically degrades RNA in DNA-RNA double strand, so that the fluorescent substance is released from the RNA and generates fluorescence (Step (4)); and
measuring the intensity of the generated fluorescence (Step (5)).

By the detection method, it is possible to determine existence and/or quantity of one or more target antigens, not only when there is only one kind of target antigen in a sample, but also when there are two or more kinds of target antigens in a sample, by a single-time performance (i.e., multiplex immunoassay).

In addition, the method may further include the following step of washing non-reacted sample, to eliminate assay-deterring substances such as RNase, between Steps (1) and (2), in order to prevent false-positive signal, thereby improving detection efficiency.

In case where the kinds of antigens to be detected are two or more, a kit can be designed such that the nucleotide sequences of the single stranded DNA oligonucleotides conjugated with detection antibodies, which specifically bind to the corresponding antigens, are different from each other and have no complementarities among the DNA oligonucleotides to avoid DNA-DNA interactions.

With the use of RNA sequences complementary to DNA oligonucleotides and with the use of different fluorescent substances labeled at the terminals of the RNA nucleotides respectively, the intensity of fluorescence varies correspondingly, depending on the existence of the particular antigens, or to their concentrations. Therefore, multiplex immunoassay for antigens of two or more kinds can be achieved with a single-time performance.

For the detection kit or the detection method, the antigens to be detected include any bioactive materials that are detectable by immunoassay. For example, the antigens may be one or more selected from the group consisting of autoantibodies, ligands, natural extracts, peptides, proteins, metal ions, synthesized medicines, natural medicines, metabolites, genomes, viruses and products by viruses, and bacteria and products by bacteria, but not be limited thereto.

The sample used for the detection of the antigens like above-whether to detect their existences or concentrations-may be used untreated or diluted in appropriated buffer solution. Following the incubation for a desired time, a further step of washing-off can be added before moving to the next step. The washing-off process removes target materials that has failed to bind to a capture antibody as well as otherwise materials that remain unbound in the sample.

For the detection kit or the detection method, the capture antibody and the detection antibody can be monoclonal or polyclonal antibodies. The capture antibody and the detection antibody are constructed to bind to the same antigen but at different sites on the antigen, so that the capture antibody and the detection antibody bind to the antigen in an uncompetitive manner and thus form a capture antibody-antigen-detection antibody complex. In order to have capture antibody and the detection antibody bind at different sites on the antigen, any technology as typically used in the relevant art can be applied, i.e., preparation of antibodies using epitopes respectively derived from differ regions on an antigen. Recently, a pair of antibodies that bind at the different sites on the same antigen has become commercially available, which can be purchased in accordance with a target antigen as desired.

It is not necessary to specifically determine the sequence of the DNA oligonucleotide conjugated with the detection antibody, because the single stranded DNA oligonucleotide acts as an intermediary for the activity of the RNase to specifically degrade RNA in a DNA/RNA double strand. That is, the DNA oligonucleotide is attached to the detection antibody bound to the target material (antigen) and binds complementarily to the fluorescence-labeled RNA oligonucleotide to form a DNA/RNA double strand, such that an RNase specifically recognizes the DNA/RNA double strand, and then specifically degrades the RNA in the DNA/RNA double strand, whereby the fluorescence at one end of the RNA is released. Therefore, it is only requested that the sequences of the DNA and the RNA are complementary, but it is not necessary to specifically define the sequences thereof.

Moreover, the DNA oligonucleotide has no limit in its length, but may include 2 to 10000, preferably 5 to 500 nucleotides for an efficient hybridization with RNA and detection efficiency. An average number of the DNA oligonucleotide linked per an antibody can be one or more. For instance, two to ten DNA oligonucleotides can be repeatedly linked. In addition, the activity of RNase H may be negatively affected if the reaction site for RNase H on the DNA oligonucleotide is too close to the antibody, and therefore, the DNA oligonucleotide may further include a base sequence repetition at 5' end, in which 5 to 50, preferably, 10 to 30 of A, T, C, or G are repeated, in addition to the region where the RNA oligonucleotide is hybridized. If the base in the repetition sequence is G, an undesired secondary structure like G-qudruplex may be caused and deter the enzyme reaction. Thus, it is more preferable that the base repetition sequence is of As, Ts or Cs, but not limited thereto.

It was noted earlier that for an immunoassay for multiple antigens, the sequences in DNA oligonucleotides-conjugated with detection antibodies capable of specific binding to coreesponding antigens-do not need to be specified. However, for efficiency in detection, it is beneficial to design the oligonucleotides to have sequences differentiated accordingly to the corresponding antigens, and to have no complementarities among the oligonucleotides such that no DNA-DNA interactions occur. In addition, the kit can be designed such that, even if complementary interactions occur between the DNA oligonucleotides having different sequences corresponding to the antigens, the DNA-DNA bindings are less stable than the complementary binding between the DNA oligonucleotide and the RNA oligonucleotide, and that the latter binding (DNA-RNA) are stronger that the former (DNA-DNA).

In an embodiment, a chemical bond (covalent bond) can be used, such as amide, disulfide, ester, ether, thioether, in order to attach a DNA oligonucleotide to a detection antibody. A cross-linker can be applied for the application of a chemical bond. One end of the cross-linker can be linked to the detection antibody through a chemical bond such as amide, disulfide, ester, ether, and thioether, while the other end of the cross-linker to the DNA oligonucleotide through a chemical bond such as amide, disulfide, ester, ether, and thioether.

Any cross-linkers conventionally used to link two biomolecues can be used. For example, cross-linkers may be one or more selected from the group including Succinimidyl-6-[β-maleimidopropionamido]hexanoate(SMPH), Succinimidyl 4-[*p*-maleimidophenyl]butyrate(SMPB), Sulfosuccinimidyl 6-(3'-[2-pyridyldithio]-propionamido)hexanoate(Sulfo-LC-SPDP), *N*-Succinimidyl{4-iodoacetyl]aminobenzoate(SIAB), but are not limited thereto.

Since DNA oligonucleotides can be linked to an antibody by chemical bonds or cross-linkers, when recognizing an antigen, the DNA oligonucleotides linked to an antigen-antibody complex by covalent bonds or cross-linkers can function to initiate enzyme reaction.

The d-Ab-DNA oligonucleotide conjugate is used in the present invention in place of a detection antibody in ELISA. As described above, the DNA oligonucleotide in the conjugate binds to an F-RNA-Q added later and acts as a template strand that makes it possible that the F-RNA-Q can be act as a substrate of RNase H.

After contacting the d-Ab-DNA oligonucleotide with the capture antibody-antigen complex to which the d-Ab-DNA oligonucleotide specifically binds, and incubating for a desired time, a further process of removing unbound d-Ab-DNA can be performed.

In the detection kit or detection method, the single stranded RNA oligonucleotide labeled with the fluorescent substance at one end and fluorescence quenching substance at the other end can be designed to have sequences complementary to the DNA sequence, so that the RNA sequence specifically binds to the DNA oligonucleotide to form a DNA/RNA double strand.

The fluorescent substance attached to the terminal of the RNA oligonucleotide can be of any type which can be used for immunoassay. The examples of the fluorescent substance include, but are not limited to, one or more selected from the group consisting of coumarin-type compounds such as 7-hydroxycoumarin, 4-methyl-7-hydroxycoumarin; xanthenes-type compounds such as ROX, TET, Texas Red, fluorescein, tetramethylrhodamine, cyanine-type compounds such as Cy3, Cy5; Bodipy-type compounds such as Bodipy FL, Bodipy 530, Bodipy R6G, Bodipy TMR, Alexa Fluor-type compounds such as Alexa Fluor 488, Alexa Fluor 647, Alexa Fluor 568, Alexa Fluor 594, Alexa Fluor 546, Alexa Fluor 350, Alexa Fluor 555, Alexa Fluor 532; and DyLight Fluor-type compounds such as DyLight 405, DyLight 488, DyLight 549, DyLight 594, DyLight 633, DyLight 649, DyLight 680, DyLight 750, DyLight 800.

The fluorescence quenching substance attached to the other terminal of the RNA oligonucleotide can be of any type, with no particular limitations. The examples of the fluorescent substance include, but are not limited to, one or more selected from the group consisting of DABCYL, QSY-type compounds (e.g., QSY-35, QSY-7, QSY-9, QSY-21), and BHQ-type compounds (e.g., BHQ-1, BHQ-2, BHQ-3). Further, it can be a fluorescent substance performing FRET(fluorescence resonance energy transfer), for example, cyan fluorescent protein(CFP), yellow fluorescent protein (YFP), green fluorescent protein(GFP). A fluorescence quenching substance to be used can be determined according to the kind of a fluorescent substance to be bound to the other terminus.

The fluorescent substance and the fluorescence quenching substance can be labeled as 5'-end and 3'-end, or 3'-end and 5'-end, respectively.

In the c-Ab-antigen-(d-Ab-DNA/F-RNA-Q) complex as formed above, the fluorescent substance (F) labeled at the one end of the single RNA strand has its fluorescence quenched due to the existence of fluorescence quenching substance labeled at the other end, proximately located to the fluorescent substance. When an RNA degradation enzyme such as RNase H is applied, the degradation activity of the RNase H against F-RNA-Q bound to d-Ab-DNA can be initiated, whereby the distance between the fluorescent substance and the fluorescence quenching substance can be distant. Therefore, amplified signals of fluorescence can be obtained after sufficient amount reaction time is allowed for RNase H. The amount of antigen bound to capture antibody is proportional to the amount of the d-Ab-DNA, and the amount of the DNA in the d-Ab-DNAs is proportional to the amplification extent of the fluorescence signals resulting from the degradation of F-RNA-Q by RNase H. Accordingly, there is a positive proportional relation between the amounts of the antigen and of the amplified fluorescence signals, enabling the quantification of the antigen via the fluorescence signals. The RNA degradation enzyme can be used along with appropriate buffer solution. The buffer solution that can be used includes what is known in the relevant field to which the present invention belongs.

In the detection kit or detection method, the RNase can be of any type that has an RNA degradation activity specific to a DNA/RNA double strand, i.e., RNase H. Particularly, RNase H is very useful for a multiplex assay using various RNA sequences, because RNase H is not sequence specific. The RNase H can be one isolated from a cell and purified, or one expressed in the form of recombinant protein and purified. The examples of RNase H include an E. coli-derived one (gene accession number: V00337, coding region: 243-707, protein accession number: CAA23620), but are not limited thereto.

The multiplex assay (multi-OLISA) according to the present invention, by which at least two antigens are detected at a time, is explained in further detail below.

The capture antibodies may be used in a form of a mixture of antibodies, each of which specifically captures its corresponding antigen in a sample including various antigens. For the performance of a multiplex-OLISA, the DNA oligonucleotides attached to the detection antibodies may be prepared so that each DNA oligonucleotide attached to a detection antibody corresponding to a certain kind of antigen has difference base sequence from that of other kinds of antigen. For example, the d-Ab-DNA conjugates can be provided as d-Ab₁-DNA₁, d-Ab₂-DNA₂, d-Ab₃-DNA₃,... d-Abₙ-DNAₙ (n is the number of the antigens to be detected). The RNAs in the F-RNA-Qs are prepared so that each of the RNAs has the base sequence complementary to that of each of the DNAs in the d-Ab-DNAs, which respectively specifically bind to the corresponding antigens. The fluorescent substances labeled on the RNAs are selected so that they can be readily distinguishable from one another, e.g., fluoresce at different wavelengths according to the corresponding antigens. An appropriate kind of fluorescence quenching substances is matched for each of the fluorescent substances. The F-RNA-Qs may be provided as F₁-RNA₁-Q₁, F₂-RNA₂-Q₂, F₃-RNA₃-Q₃, .... Fₙ-RNAₙ-Qₙ (n is the number of the antigens to be detected). It may also be possible to quench the fluorescence from more than one fluorescent substance using only one fluorescence quenching substance.

For the multiplex-OLISA, n kinds of fluorescence signals generated and amplified from the Fₙ-RNAₙ-Qₙs by RNA degradation by RNase H have difference wavelengths from one another, and thus, each of them can be detected at its appropriate wavelength. Each of the capture antibodies binds to its corresponding antigen; a d-Ab-DNA, where the detection antibody is specific to the antigen, binds thereto; and subsequently, an F-RNA-Q, where the RNA sequence is complementary to the DNA, binds to its d-Ab-DNA, resulting in the formation of complexes each of which respectively correspond to the antigens to be detected. Consequently, the fluorescence intensities obtained by RNase H reaction are respectively proportional to the amounts of the corresponding antigens. Therefore, by using the method provided above, multiple antigens in the same sample can be detected and quantified through a multiplex-OLISA where the fluorescence signals are generated and amplified by RNase H.

The conventional ELISA methods have required a different enzyme for each of the antigen to be detected to perform a multiplex assay. In addition, unless an enzyme used is exclusively specific to a target antigen, other antigens tend to act as competitive substrates, making it difficult to achieve specificity and accuracy in detection. However, the present invention provides DNA oligonucleotides each of which has different sequence according to the kind of antigens, the RNA oligonucleotides respectively complementary to the DNA oligonucleotides, and RNase H degrading a DNA/RNA double strand with no sequence specificity, such that multiple antigens can be simultaneously detected with a single performance of assay irrespective of the number of target antigens.

While ELISA immunoassay is one of the most commonly used immunoassays in the field of antigen detection and medical diagnostics, there is a difficult for an observer to compare comparative amounts of multiple substances in the same sample, since ELISA-based multiplex assay requires separate performance of analytic processes for individual substances. Further, it has recently known that diagnostically difficult diseases such as cancer and Alzheimer disease can be diagnosed not by a single marker. More precise diagnosis is possible only through the analysis of relative relationships resulting from increases/decreases in various relevant proteins and physiological materials. For the profiling of such multiple markers in the same sample, a multiplex ELISA must be used, by which the multiple markers can be simultaneously analyzed in a single microplate well. While the conventional multiplex ELISA utilizes more than one pairs of enzyme-substrate, the multiplex-OLISA method and the kit for the same according to the present invention can perform a multiplex assay with only a single kind of enzyme, with using the identical antibody-coated microwell plate and the identical analytic tool for microwell plate using fluorescence or absorption as used in the conventional ELISA. Therefore, a simpler and more cost-effective method is provided by the present invention. In this respect, the multiplex-OLISA according to the present invention can contribute to a situation in which multiple antigens have to be detected simultaneously, and particularly to the field of disease diagnostics where multiple diagnostic markers have to be simultaneously analyzed.

### EXAMPLES

The present invention is further explained in more detail with reference to the following examples. These examples, however, should not be interpreted as limiting the scope of the present invention in any manner.

### <Example 1 > OLISA analysis using RNase H for a single antigen

The detection of Alpha-fetoprotein (AFP, human fetal cord serum derived, Fitzgerald, Inc.) was performed as follows.

### 1.1. Preparation of detection probe (d-Ab-DNA)

50 µℓ of 3.4 mg/mL monoclonal antibody (1) against AFP, as provided by Fitzgerald Inc. in a pair for sandwich ELISA (Cat# 10C-CR1007M5, recognizing AFP from cord blood), was mixed with 50 µℓ of PBS buffer and 1 *µ*ℓ of 100 mM SMPH (succnimidyl-6-[β-maleimidopropionamido]hexanoate), and incubated at room temperature for 30 minutes. The resultant solution was diluted in 15 mL of PBS buffer and centrifuged at 4,000 rpm for 40 minutes at 4°C using Amicon Ultra-15 (Ultracel-30K)(Millipore). The process was repeated three times and the supernatant ① was obtained.

At the same time, 100 µl of 100 µM DNA solution (the sequence of the DNA nucleotide being 5'- TTTTTTTTTTTTTTTTTTTTTAACCACGT-3, SEQ ID NO: 1, wherein 20 thymidines are added at 5' end of the DNA oligonucleotide in order to avoid an inhibiting effect on reaction of RNase by the attachment of the DNA oligonucleotide to the antibody) and 15 µℓ of 1 M dithiothreitol (DTT) were mixed and incubated for 15 minutes at room temperature. Then addition and removal of 100 µℓ ethyl acetate were repeated five times. Residual DDT and thiol fragments were removed using a MicroSpin G-25 column. The remaining DNA solution and the supernatant ① obtained above were mixed and incubated at room temperature for 30 minutes. The resultant solution was diluted in 15 mL PBS buffer and was centrifuged four times using Amicon Ultra-15 (Ultracel-30K) at 4,000 rpm for 40 minutes, at 4°C. The resulting supernatant is the d-Ab-DNA conjugate (I), in which the monoclonal antibody (1) and the DNA fragment are conjugated. The d-Ab-DNA conjugate (I) was used as a detection probe in the OLISA analysis.

### 1.2. The preparation of capture antibody and RNA probe, and OLISA analysis

The monoclonal antibody (2) against AFP, as provided by Fitzgerald Inc. in a pair for sandwich ELISA (Cat# 10C-CR1007M4, recognizing AFP from cord blood) was diluted in PBS buffer at a concentration of 10 µg/mL. The prepared solution was added in the 96-well microplate (100 µℓ per well) and placed at 4°C for 15 hours. After three times of washing with 200 µℓ PBS buffer, 200 *µ*ℓ of 3%(w/v) bovine serum albumin(BSA)/PBS solution was added. The microplate was then incubated at room temperate for 2 hours. After three times of washing with 200µℓ of PBST (PBS + 0.05%(w/v) Tween-20), the antigens (AFP) were diluted in 3%(w/v) BSA/PBS solution at various concentrations and then, incubated in the 96-well microplate for two hours (100 *µ*ℓ per well).

Subsequently, the wells were washed three times with 200 µℓ of PBST. The d-Ab-DNA conjugate (I) solution as prepared in Example 1.1 was diluted in 3%(w/v) BSA/PBS at 1:30 ratio, added to the wells in the amount of 100µℓ per a well, and incubated for 2 hours at room temperature.

The well was washed three times with 200 µℓ of PBST solution. Then 100 µℓ of reaction solution containing 40 mM Tris-HCl, 4 mM MgCl₂, 1 mM DTT, 0.003% BSA, 400 nM fluorescein(FAM)-RNA1-dabcyl, 0.1 U Protector RNase Inhibitor (Roche Inc. Cat. # 03335402001), and 6 U RNase H (Takara bio Inc. Code No. 2150A) was added to the wells and incubated at room temperature for 2 hours. The oligonucleotide sequence of fluorescein(FAM)-RNA1-dabcyl is FAM-5'-CACUGUGGUU(SEQ ID NO: 2)-3'-dabcyl. After the reaction was completed, fluorescence intensity was measured using the microplate reader (Varioskan flash, Thermoscientific Inc.) at excitation and absorption wavelengths of 485±10 nm and 535±10 nm, respectively.

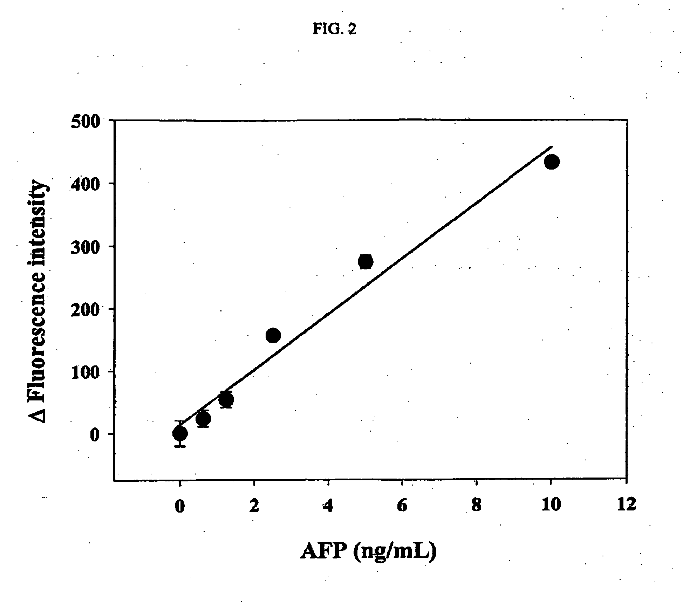

The result is shown in Fig. 2. As shown in Fig. 2, the intensity of fluorescence increases proportionally to the concentrations of target material AFP. That is, AFP was efficiently detected with the method in the present example.

### <Example 2> Multiplex-OLISA analysis using RNase H for simultaneous analysis of two kinds of antigens coexisting in a same sample

The following experiment was performed for simultaneous detection of Alpha-fetoprotein (AFP) and prostate specific antigen (PSA, Fitzgerald, Cat # 30C-CP1017U).

### 2.1. Preparation of detection probe (d-Ab-DNA)

The detection probe for AFP was prepared using the same manner as described in Example 1.1

The detection probe for PSA was prepared in the following manner. 50 µℓ of the antibody solution at the concentration of 2.25 mg/mL was mixed with 50 µℓ of PBS buffer solution and 1 µℓ of 100 mM SMPH (succnimidyl-6-[β-maleimidopropionamido]hexanoate). The antibody solution contains the monoclonal antibody (3) against PSA, as provided by Fitzgerald Inc. in pair for total PSA analysis in sandwich ELISA, Cat# 10-P20D. The mixture was allowed for reaction at room temperature for 30 minutes. The resultant solution was diluted in 15 mL PBS buffer solution and centrifuged using Amicon Ultra-15 (Ultracel-30K) at 4,000 rpm for 40 minutes, at 4°C. The process was repeated three times, and supernatant (②) was obtained.

At the same time, 100 µℓ of 100 µM DNA solution (the sequence of the DNA nucleotide being 5'-ITTTTTTTTTTTTTTTTTTTACTCTATGGG-3', SEQ ID NO: 3) and 15 µℓ of 1 M dithiothreitol (DTT) were mixed together and allowed for reaction for 15 minutes at room temperature. Then addition and removal of 100 µℓ ethyl acetate were repeated five times. Residual DDT and thiol fragments were then removed using a MicroSpin G-25 column. The remaining DNA solution and the supernatant ② obtained in the above were mixed together and were allowed for reaction for 30 minutes. The resultant solution was diluted in 15 mL PBS buffer solution and centrifuged four times using Amicon Ultra-15 (Ultracel-30K) at 4,000 rpm for 40 minutes, at 4°C. The resulting supernatant is the d-Ab-DNA conjugate (II), in which the monoclonal antibody (3) and the DNA fragment was conjugated. The d-Ab-DNA conjugate (II) was used as a detection probe for PSA in the OLISA analysis.

### 2.2. The preparation of capture probe and RNA conjugate and OLISA analysis

The monoclonal antibody (2) against AFP (Example 1.2) and the monoclonal antibody (4) (Fitzgerald Inc., Catalog # 10-P20E) against PSA were dissolved in PBS buffer, each at the concentration of 10 µg/mL. The prepared solution was added in the 96-well microplate (100 µℓ per well) and placed at 4°C for 15 hours. After three times of washing with 200 µℓ PBS buffer, 200 µℓ of 3%(w/v) bovine serum albumin(BSA)/PBS solution was added. The microplate was then incubated at room temperate for 2 hours. After three times of washing with 200*µ*ℓ of PBST (PBS + 0.05%(w/v) Tween-20), each of the antigens (AFP(Fitzgerald), PSA(Fitzgerald)) was diluted in 3%(w/v) BSA/PBS solution at various concentrations and placed in the 96-well microplate for two hours (100 µℓ per a well) at room temperature.

Subsequently, the wells were washed three times with 200µℓ of PBST. The d-Ab-DNA conjugates corresponding to the antigens as prepared in Example 2.1 were diluted in 3%(w/v) BSA/PBS at 1:30 ratio and placed in the wells (100µℓ per well) for 2 hours at room temperature. The microplate was washed three times with 200µℓ of PBST solution. Then 100 µℓ of the reaction solution, which contains 40 mM Tris-HCl, 4 mM MgCl2, 1 mM DTT, 0.003% BSA, 400 nM fluorescein-RNA1-dabcyl, 400 nM ROX-RNA2-BHQ2 (ROX-5'-CCCAUAGAGU (SEQ ID NO: 4)-3'-BHQ2), 0.1 U Protector RNase Inhibitor (Roche Inc. Cat. # 03335402001), and 6 U RNase H (Takara bio Inc. Code No. 2150A), was added in the well for reaction at room temperature for 1 hours. After the reaction was completed, fluorescence intensity was measured using a microplate reader (Varioskan flash, Thermoscientific Inc.) at emission wavelengths of 535±10 n m and 589±5 nm for fluorescein and ROX, respectively.

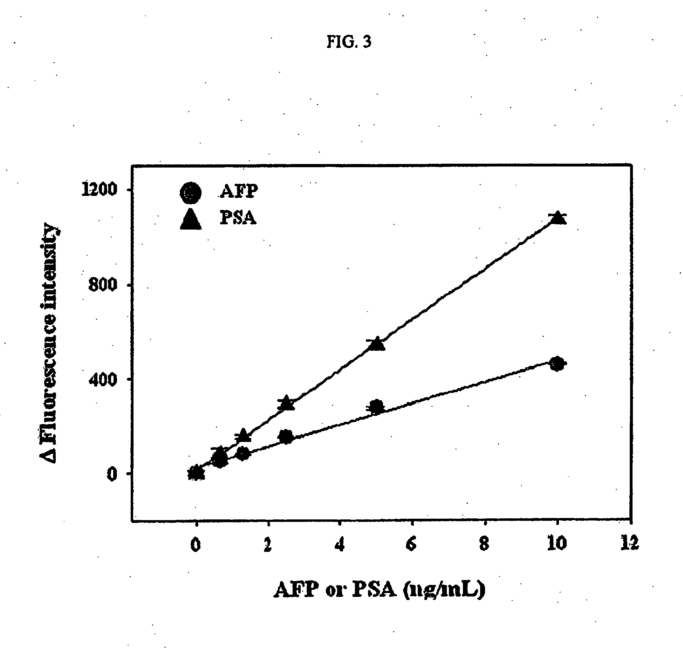

The result obtained is shown in Fig. 3. As shown in Fig. 3, the intensity of fluorescence increases proportionally to the concentrations of AFP and PSA, the target substances. That is, AFP and PSA were efficiently detected without being interfered by the existence of each other, by the method of the present example.

## Claims

1. An antigen detection kit comprising:
- a solid support;
a capture antibody immobilized on said support, wherein the capture antibody capable of specifically binding to an antigen;
a detection antibody-DNA conjugate (d-Ab-DNA conjugate) comprising a detection antibody (d-Ab) capable of specifically binding to the antigen at a different site of the antigen from the site to which the capture antibody binds, and a single stranded DNA oligonucleotide;
a fluorescent substance-RNA-a fluorescence quenching substance conjugate, comprising a single stranded RNA oligonucleotide complementary to the DNA oligonucleotide, a fluorescent substance bound to one terminus of the single stranded RNA oligonucleotide, and a fluorescence quenching substance bound to the other terminus of the single stranded RNA oligonucleotide; and
- RNase H.

2. The antigen detection kit according to Claim 1, wherein said antigen is one or more selected from the group consisting of autoantibodies, ligands, natural extracts, peptides, proteins, metal ions, synthesized medicines, natural medicines, metabolites, genomes, viruses and products by viruses, and bacteria and products by bacteria.

3. The antigen detection kit according to Claim 2, wherein the antigen is two or more selected from the group consisting of autoantibodies, ligands, natural extracts, peptides, proteins, metal ions, synthesized medicines, natural medicines, metabolites, genomes, viruses and products by viruses, and bacteria and products by bacteria, and the kit is capable of simultaneously detecting two or more kinds of antigens.

4. The antigen detection kit according to Claim 3, wherein the detection antibody-DNA conjugates have different DNA oligonucleotides from one another corresponding to the kinds of the antigens to be detected, and the DNA oligonucleotides are non-complementary to one another.

5. The antigen detection kit according to any one of Claims 1 through 4, wherein the detection antibody and the DNA oligonucleotide is bound through a chemical bond of one or more selected from the group amide, disulfide, ester, ether, and thioether.

6. The antigen detection kit according to Claim 5, wherein the detection antibody and the DNA oligonucleotide is bound through one or more cross-linkers selected from the group consisting SMPH (Succinimidyl-6-[[beta]-maleimidopropionamido]hexanoate), SMPB (Succinimidyl 4-[p-maleimidophenyl]butyrate), Sulfo-LC-SPDP (Sulfosuccinimidyl 6-(3'-[2-pyridyldithio]-propionamido)hexanoate), and SIAB (N-Succinimidyl[4-iodoacetyl]aminobenzoate).

7. The antigen detection kit according to any one of Claims 1 through 4, wherein the fluorescent substance is one or more selected from the group consisting of coumarin group, xanthenes group, cyanine group, Bodipy group, Alexa Fluor group, and DyLight Fluor group fluorescent substances.

8. The antigen detection kit according to any one of Claims 1 through 4, wherein the fluorescence quenching substance is one or more selected from the group consisting of dabcyl group substances, QSY group substances, BHQ group substances, and a fluorescent substance performing FRET(fluorescence resonance energy transfer).

9. An antigen detection method comprising the steps of:
- contacting a sample with a capture antibody capable of specifically binding to an antigen to be detected, to form an antigen-antibody binding;
- contacting a detection antibody-DNA oligonucleotide (d-Ab-DNA) conjugate with the antigen bound to the capture antibody, wherein the detection antibody-DNA oligonucleotide conjugate comprises a detection antibody (d-Ab) capable of specifically binding to the antigen at a different site of antigen from the site to which the capture antibody binds, and a single stranded DNA oligonucleotide that is bound to a detection antibody, to form a biding between the antigen and the d-Ab-DNA conjugate;
- contacting a fluorescent substance-RNA-fluorescence quenching substance (F-RNA-Q) conjugate with the antigen bound both to the capture antibody and to the d-Ab-DNA conjugate, wherein the F-RNA-Q conjugate comprises a single stranded RNA oligonucleotide complementary to the DNA oligonucleotide, a fluorescent substance at one terminus of the RNA oligonucleotide, and a fluorescence quenching substance at the other terminus of the RNA oligonucleotide, to form a DNA-RNA double strand through the hybridization of DNA-RNA;
- treating the obtained DNA-RNA double strand with an RNase that degrades the RNA in the DNA-RNA double strand, to release the fluorescent substance from the RNA and generate fluorescence; and
- measuring the intensity of the generated fluorescence.

10. The antigen detection method according to claim 9, wherein the antigen is one or more selected from the group consisting of autoantibodies, ligands, natural extracts, peptides, proteins, metal ions, synthesized medicines, natural medicines, metabolites, genomes, viruses and products by viruses, and bacteria and products by bacteria.

11. The antigen detection method according to claim 9, wherein the antigen is two or more selected from the group consisting of autoantibodies, ligands, natural extracts, peptides, proteins, metal ions, synthesized medicines, natural medicines, metabolites, genomes, viruses and products by viruses, and bacteria and products by bacteria, and the method is capable of simultaneously detecting two or more kinds of antigens by using tow or more RNA oligonucleotides each of which is bound to a different fluorescent substance corresponding to the kinds of the antigens.

12. The antigen detection method according to claim 11, wherein the detection antibody-DNA conjugates have different DNA oligonucleotides from one another corresponding to the kinds of antigens to be detected, and the DNA oligonucleotides are non-complementary to one another.

13. The antigen detection method according to any one of Claims 9 through 12, wherein the fluorescent substance is one or more selected from the group consisting of coumarin group, xanthenes group, cyanine group, Bodipy group, Alexa Fluor group, and DyLight Fluor group fluorescent substances.

14. The antigen detection method according to any one of Claims 9 through 12, wherein the fluorescence quenching substance is one or more selected from the group consisting of dabcyl group substances, QSY group substances, BHQ group substances, and a fluorescent substance performing FRET(fluorescence resonance energy transfer).

## Patentansprüche

1. Antigen-Nachweis-Kit, umfassend:
- einen festen Träger;
einen an dem Träger immobilisierten Capture-Antikörper, wobei der Capture-Antikörper fähig ist, spezifisch an ein Antigen zu binden;
ein Nachweisantikörper-DNA-Konjugat (d-Ab-DNA-Konjugat), umfassend einen Nachweisantikörper (d-Ab), der fähig ist, spezifisch an das Antigen zu binden und zwar an einer Stelle des Antigens, die von der Stelle, an welcher der Capture-Antikörper bindet, verschieden ist, und ein einzelsträngiges DNA-Oligonukleotid;
ein fluoreszierende Substanz-RNA-Fluoreszenz-Quenchersubstanz-Konjugat, umfassend ein einzelsträngiges RNA-Oligonukleotid, das zu dem DNA-Oligonukleotid komplementär ist, eine fluoreszierende Substanz, die an einen Terminus des einzelsträngigen RNA-Oligonukleotids gebunden ist, und eine Fluoreszenz-Quenchersubstanz, die an den anderen Terminus des einzelsträngigen RNA-Oligonukleotids gebunden ist; und
- RNase H.

2. Antigen-Nachweis-Kit nach Anspruch 1, wobei es sich bei dem Antigen um eines oder mehrere handelt, das/die aus der Gruppe ausgewählt ist/sind, die aus Autoantikörpern, Liganden, natürlichen Extrakten, Peptiden, Proteinen, Metallionen, synthetisch hergestellten Arzneimitteln, natürlichen Arzneimitteln, Stoffwechselprodukten, Genomen, Viren und Produkten von Viren, Bakterien und Produkten von Bakterien besteht.

3. Antigen-Nachweis-Kit nach Anspruch 2, wobei es sich bei dem Antigen um zwei oder mehr handelt, die aus der Gruppe ausgewählt sind, die aus Autoantikörpern, Liganden, natürlichen Extrakten, Peptiden, Proteinen, Metallionen, synthetisch hergestellten Arzneimitteln, natürlichen Arzneimitteln, Stoffwechselprodukten, Genomen, Viren und Produkten von Viren, Bakterien und Produkten von Bakterien besteht, und das Kit fähig ist, zwei oder mehr Arten von Antigenen gleichzeitig nachzuweisen.

4. Antigen-Nachweis-Kit nach Anspruch 3, wobei die Nachweisantikörper-DNA-Konjugate voneinander verschiedene DNA-Oligonukleotide aufweisen, die den Arten der nachzuweisenden Antigene entsprechen, wobei die DNA-Oligonukleotide nicht komplementär zueinander sind.

5. Antigen-Nachweis-Kit nach einem der Ansprüche 1 bis 4, wobei der Nachweisantikörper und das DNA-Oligonukleotid chemisch gebunden sind und zwar durch eine chemische Bindung von einer oder mehreren, ausgewählt aus der Gruppe der Amid-, Disulfid-, Ester-, Ether- und Thioetherbindungen.

6. Antigen-Nachweis-Kit nach Anspruch 5, wobei der Nachweisantikörper und das DNA-Oligonukleotid durch einen oder mehrere Vernetzer gebunden sind, der/die aus der Gruppe ausgewählt ist/sind, die aus SMPH (Succinimidyl-6-[[beta]-maleimidpropionamido]hexanoat), SMPB (Succinimidyl-4-[p-maleimidphenyl]butyrat), Sulfo-LC-SPDP (Sulfosuccinimidyl-6-(3'-[2-pyridyldithio]-propionamido)hexanoat) und SIAB (N-Succinimidyl[4-iodacetyl]aminobenzoat) besteht.

7. Antigen-Nachweis-Kit nach einem der Ansprüche 1 bis 4, wobei es sich bei der fluoreszierenden Substanz um eine oder mehrere handelt, die aus der Gruppe ausgewählt ist/sind, die aus Cumarinen, Xanthenen, Cyaninen, Bodipy-, Alexa Fluor- und DyLight Fluor-Fluoreszenzfarbstoffen besteht.

8. Antigen-Nachweis-Kit nach einem der Ansprüche 1 bis 4, wobei es sich bei der Fluoreszenz-Quenchersubstanz um eine oder mehrere handelt, die aus der Gruppe ausgewählt ist/sind, die aus Dabcyl-, QSY-, BHQ-Substanzen und einer einen FRET (Fluoreszenz-Resonanzenergietransfer) ausführenden fluoreszierenden Substanz besteht.

9. Antigen-Nachweisverfahren, umfassend die Schritte:
- Inkontaktbringen einer Probe mit einem Capture-Antikörper, der fähig ist, spezifisch an ein nachzuweisendes Antigen zu binden, um eine Antigen-Antikörper-Bindung auszubilden;
- Inkontaktbringen eines Nachweisantikörper-DNA-Oligonukleotid- (d-Ab-DNA-) Konjugats mit dem am Capture-Antikörper gebundenen Antigen, wobei das Nachweisantikörper-DNA-Oligonukleotid-Konjugat einen Nachweisantikörper (d-Ab), der fähig ist, spezifisch an das Antigen zu binden und zwar an einer Stelle des Antigens, die von der Stelle, an welcher der Capture-Antikörper bindet, verschieden ist, und ein einzelsträngiges DNA-Oligonukleotid, das an einen Nachweisantikörper gebunden ist, umfasst, um zwischen dem Antigen und dem d-Ab-DNA-Konjugat eine Bindung auszubilden;
- Inkontaktbringen eines fluoreszierende Substanz-RNA-Fluoreszenz-Quenchersubstanz- (F-RNA-Q-) Konjugats mit dem Antigen, das sowohl am Capture-Antikörper als auch am d-Ab-DNA-Konjugat gebunden ist, wobei das F-RNA-Q-Konjugat ein einzelsträngiges RNA-Oligonukleotid, das zu dem DNA-Oligonukleotid komplementär ist, eine fluoreszierende Substanz an einem Terminus des RNA-Oligonukleotids und eine Fluoreszenz-Quenchersubstanz am anderen Terminus des RNA-Oligonukleotids umfasst, um durch die DNA-RNA-Hybridisierung einen DNA-RNA-Doppelstrang zu bilden;
- Behandeln des erhaltenen DNA-RNA-Doppelstrangs mit einer RNase, welche die RNA in dem DNA-RNA-Doppelstrang abbaut, um die fluoreszierende Substanz aus der RNA freizusetzen und Fluoreszenz zu erzeugen; und
- Messen der Intensität der erzeugten Fluoreszenz.

10. Antigen-Nachweisverfahren nach Anspruch 9, wobei es sich bei dem Antigen um eines oder mehrere handelt, das/die aus der Gruppe ausgewählt ist/sind, die aus Autoantikörpern, Liganden, natürlichen Extrakten, Peptiden, Proteinen, Metallionen, synthetisch hergestellten Arzneimitteln, natürlichen Arzneimitteln, Stoffwechselprodukten, Genomen, Viren und Produkten von Viren, Bakterien und Produkten von Bakterien besteht.

11. Antigen-Nachweisverfahren nach Anspruch 9, wobei es sich bei dem Antigen um zwei oder mehr handelt, die aus der Gruppe ausgewählt sind, die aus Autoantikörpern, Liganden, natürlichen Extrakten, Peptiden, Proteinen, Metallionen, synthetisch hergestellten Arzneimitteln, natürlichen Arzneimitteln, Stoffwechselprodukten, Genomen, Viren und Produkten von Viren, Bakterien und Produkten von Bakterien besteht, und das Verfahren fähig ist, zwei oder mehr Arten von Antigenen gleichzeitig nachzuweisen, indem zwei oder mehr RNA-Oligonukleotide verwendet werden, wovon jedes an eine andere fluoreszierende Substanz, entsprechend der Arten der Antigene, gebunden ist.

12. Antigen-Nachweisverfahren nach Anspruch 11, wobei die Nachweisantikörper-DNA-Konjugate voneinander verschiedene DNA-Oligonukleotide aufweisen, die den Arten der nachzuweisenden Antigene entsprechen, wobei die DNA-Oligonukleotide nicht komplementär zueinander sind.

13. Antigen-Nachweisverfahren nach einem der Ansprüche 9 bis 12, wobei es sich bei der fluoreszierenden Substanz um eine oder mehrere handelt, die aus der Gruppe ausgewählt ist/sind, die aus Cumarinen, Xanthenen, Cyaninen, Bodipy-, Alexa Fluor- und DyLight Fluor-Fluoreszenzfarbstoffen besteht.

14. Antigen-Nachweisverfahren nach einem der Ansprüche 9 bis 12, wobei es sich bei der Fluoreszenz-Quenchersubstanz um eine oder mehrere handelt, die aus der Gruppe ausgewählt ist/sind, die aus Dabcyl-, QSY-, BHQ-Substanzen und einer einen FRET (Fluoreszenz-Resonanzenergietransfer) ausführenden fluoreszierenden Substanz besteht.

## Revendications

1. Kit de détection d'antigène comprenant :
- un support solide ;
un anticorps de capture immobilisé sur ledit support, où l'anticorps de capture est capable de se lier spécifiquement à un antigène ;
un conjugué anticorps de détection-ADN (conjugué d-Ab-ADN) comprenant un anticorps de détection (d-Ab) capable de se lier spécifiquement à l'antigène au niveau d'un site différent de l'antigène à partir du site auquel se lie l'anticorps de capture, et un oligonucléotide d'ADN simple brin ;
un conjugué substance fluorescente-ARN-substance d'extinction de fluorescence, comprenant un oligonucléotide d'ARN simple brin complémentaire à l'oligonucléotide d'ADN, une substance fluorescente liée à une extrémité de l'oligonucléotide d'ARN simple brin, et une substance d'extinction de fluorescence liée à l'autre extrémité de l'oligonucléotide d'ARN simple brin ; et
- une RNase H.

2. Kit de détection d'antigène selon la revendication 1, dans lequel ledit antigène est un ou plusieurs élément(s) choisi(s) dans le groupe constitué d'auto-anticorps, de ligands, d'extraits naturels, de peptides, de protéines, d'ions métalliques, de médicaments synthétiques, de médicaments naturels, de métabolites, de génomes, de virus et de produits provenant de virus, et de bactéries et de produits provenant de bactéries.

3. Kit de détection d'antigène selon la revendication 2, dans lequel l'antigène représente deux éléments ou plus choisis dans le groupe constitué d'auto-anticorps, de ligands, d'extraits naturels, de peptides, de protéines, d'ions métalliques, de médicaments synthétiques, de médicaments naturels, de métabolites, de génomes, de virus et de produits provenant de virus, et de bactéries et de produits provenant de bactéries, et le kit est capable de détecter simultanément deux types ou plus d'antigènes.

4. Kit de détection d'antigène selon la revendication 3, dans lequel les conjugués anticorps de détection-ADN ont des oligonucléotides d'ADN différents les uns des autres correspondant aux types des antigènes à détecter, et les oligonucléotides d'ADN sont non complémentaires les uns aux autres.

5. Kit de détection d'antigène selon l'une quelconque des revendications 1 à 4, dans lequel l'anticorps de détection et l'oligonucléotide d'ADN sont liés par une liaison chimique d'un ou de plusieurs élément(s) choisi(s) dans le groupe d'amide, de disulfure, d'ester, d'éther, et de thioéther.

6. Kit de détection d'antigène selon la revendication 5, dans lequel l'anticorps de détection et l'oligonucléotide d'ADN sont liés par un ou plusieurs agent(s) de réticulation choisi(s) dans le groupe constitué de SMPH (6-[[bêta]-maléimidopropionamido]hexanoate de succinimidyle), de SMPB (4-[p-maléimidophényl]butyrate de succinimidyle), de Sulfo-LC-SPDP (6-(3'-[2-pyridyldithio]-propionamido)hexanoate de Sulfosuccinimidyl), et de SIAB (N-succinimidyl[4-iodoacétyl]aminobenzoate).

7. Kit de détection d'antigène selon l'une quelconque des revendications 1 à 4, dans lequel la substance fluorescente est une ou plusieurs substance(s) choisie(s) dans le groupe constitué de substances fluorescentes de groupe coumarine, de groupe xanthènes, de groupe cyanine, de groupe Bodipy, de groupe Alexa Fluor, et de groupe DyLight Fluor.

8. Kit de détection d'antigène selon l'une quelconque des revendications 1 à 4, dans lequel la substance d'extinction de fluorescence est une ou plusieurs substance(s) choisie(s) dans le groupe constitué de substances de groupe dabcyl, de substances de groupe QSY, de substances de groupe BHQ, et d'une substance fluorescente effectuant un FRET (transfert d'énergie de fluorescence par résonance).

9. Procédé de détection d'antigène comprenant les étapes qui consistent :
- à mettre en contact un échantillon avec un anticorps de capture capable de se lier spécifiquement à un antigène à détecter, pour former une liaison antigène-anticorps ;
- à mettre en contact un conjugué anticorps de détection-oligonucléotide d'ADN (d-Ab-ADN) avec l'antigène lié à l'anticorps de capture, où le conjugué anticorps de détection-oligonucléotide d'ADN comprend un anticorps de détection (d-Ab) capable de se lier spécifiquement à l'antigène au niveau d'un site différent d'antigène à partir du site auquel se lie l'anticorps de capture, et un oligonucléotide d'ADN simple brin qui est lié à un anticorps de détection, pour former une liaison entre l'antigène et le conjugué d-Ab-ADN ;
- à mettre en contact un conjugué substance fluorescente-ARN-substance d'extinction de fluorescence (F-ARN-Q) avec l'antigène lié à la fois à l'anticorps de capture et au conjugué d-Ab-ADN, où le conjugué F-ARN-Q comprend un oligonucléotide d'ARN simple brin complémentaire à l'oligonucléotide d'ADN, une substance fluorescente au niveau d'une extrémité de l'oligonucléotide d'ARN, et une substance d'extinction de fluorescence au niveau de l'autre extrémité de l'oligonucléotide d'ARN, pour former de l'ADN-ARN double brin par l'hybridation d'ADN-ARN ;
- à traiter l'ADN-ARN double brin obtenu avec une RNase qui dégrade l'ARN dans l'ADN-ARN double brin, pour libérer la substance fluorescente de l'ARN et pour générer une fluorescence ; et
- à mesurer l'intensité de la fluorescence générée.

10. Procédé de détection d'antigène selon la revendication 9, dans lequel l'antigène est un ou plusieurs élément(s) choisi(s) dans le groupe constitué d'auto-anticorps, de ligands, d'extraits naturels, de peptides, de protéines, d'ions métalliques, de médicaments synthétiques, de médicaments naturels, de métabolites, de génomes, de virus et de produits provenant de virus, et de bactéries et de produits provenant de bactéries.

11. Procédé de détection d'antigène selon la revendication 9, dans lequel l'antigène représente deux éléments ou plus choisis dans le groupe constitué d'auto-anticorps, de ligands, d'extraits naturels, de peptides, de protéines, d'ions métalliques, de médicaments synthétiques, de médicaments naturels, de métabolites, de génomes, de virus et de produits provenant de virus, et de bactéries et de produits provenant de bactéries, et le procédé est capable de détecter simultanément deux types ou plus d'antigènes en utilisant deux oligonucléotides d'ARN ou plus dont chacun est lié à une substance fluorescente différente correspondant aux types des antigènes.

12. Procédé de détection d'antigène selon la revendication 11, dans lequel les conjugués anticorps de détection-ADN ont des oligonucléotides d'ADN différents les uns des autres correspondant aux types d'antigènes à détecter, et les oligonucléotides d'ADN sont non complémentaires les uns aux autres.

13. Procédé de détection d'antigène selon l'une quelconque des revendications 9 à 12, dans lequel la substance fluorescente est une ou plusieurs substance(s) choisie(s) dans le groupe constitué de substances fluorescentes de groupe coumarine, de groupe xanthènes, de groupe cyanine, de groupe Bodipy, de groupe Alexa Fluor, et de groupe DyLight Fluor.

14. Procédé de détection d'antigène selon l'une quelconque des revendications 9 à 12, dans lequel la substance d'extinction de fluorescence est une ou plusieurs substance(s) choisie(s) dans le groupe constitué de substances de groupe dabcyl, de substances de groupe QSY, de substances de groupe BHQ, et d'une substance fluorescente effectuant un FRET (transfert d'énergie de fluorescence par résonance).
